# EUROPEAN PATENT SPECIFICATION

(11) **EP 2 211 800 B1**
(45) Date of publication and mention of the grant of the patent: **02.05.2012**
(21) Application number: 08841283.8
(22) Date of filing: 21.10.2008
(51) Int. Cl.: A61F 9/007, A61M 1/00

(54) **DEVICE FOR STABILIZATION OF EYE FRONT CHAMBER DURING OPHTALMIC SURGERY WITH HIGH VACUUM SUCKING**
VORRICHTUNG ZUR STABILISIERUNG DER AUGENVORDERKAMMER WÄHREND EINER AUGENOPERATION MIT HOCHVAKUUM-SAUGUNG
DISPOSITIF PERMETTANT DE STABILISER LA CHAMBRE FRONTALE DE L' IL PENDANT UNE CHIRURGIE OPHTALMIQUE AVEC UNE FORTE ASPIRATION SOUS VIDE

(30) Priority: 25.10.2007 IT RM20070559
(43) Date of publication of application: 04.08.2010
(73) Proprietor: Optikon 2000 S.p.A., 00138 Roma (IT)
(72) Inventor: KAWANO, Koji, KAGOSHIMA-KEN 895-0023 (JP); KITSUKAWA, Hiroyuki, Tokyo (JP); LONGO, Dario, I-00069 Trevignano Romano (RM) (IT); ANGELINI, Giampiero, I-00015 Monterotondo (RM) (IT)
(74) Representative: Iannone, Carlo Luigi
(86) International application number: PCT/IT2008/000658
(87) International publication number: WO 2009/054019

(56) References cited:
- EP-A- 0 862 902
- US-A- 4 340 037
- US-A1- 2002 095 113
- US-A1- 2006 135 974

## Description

The present invention relates to a device for stabilization of eye front chamber during ophthalmic surgery with high vacuum aspiration.

More specifically, the invention concerns a device for hydrodynamic stabilization of the eye front chamber and for preventing collapses of cornea during ophthalmic surgery including a reservoir with elastic walls along the irrigation line, connected to the irrigation inlet of ultrasound handpiece or Irrigation - Aspiration.

As it is well known, maintaining proper IntraOcular Pressure (IOP) is really important during ophthalmic surgery. Particularly, during cataract removal interventions by ultrasound scalpel (phacoemulsification), a pump, connected to the handpiece aspiration line, removes liquids and nucleus fragments from eye while an irrigation line, supplied by a balanced saline solution reservoir and connected to the surgical handpiece, replaces sucked liquids and maintains stable IOP.

Most recent phacoemulsification techniques require, for a higher efficiency, high aspiration vacuum and flow levels. Consequently, irrigation source will have to be operated at high pressures.

For example, a solution is described in US patent n° 6,042,586 concerning an "Anti-cornea-collapsing device for ophthalmic surgery using ultra-high vacuum aspiration", using a very high vacuum aspiration.

While making the surgical intervention, aspiration line can instantaneously clog due to a nucleus fragment not emulsified as yet, instantaneously interrupting aspiration flow. Flow along the irrigation line will prosecute by inertia for a short time, causing a temporaneous but meaningful increase of IOP.

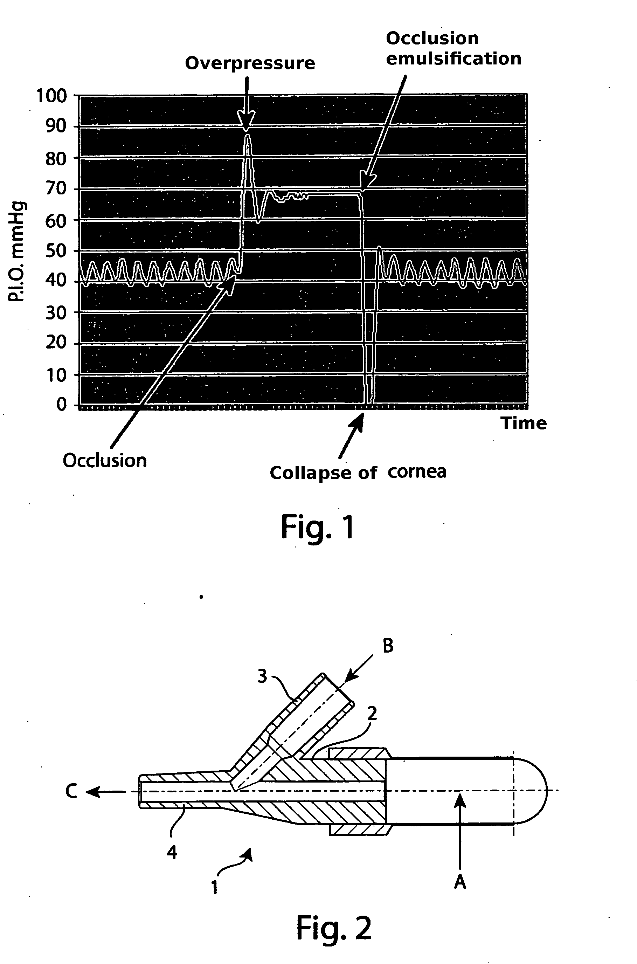

When said nucleus fragment will be emulsified, aspiration flow will instantaneously start again. Due to inertia, irrigation flow will start again with a certain delay, causing a meaningful dropping of IOP (see figure 1 of the enclosed drawings) that could even cause a cornea collapse, with serious damages for patient.

Further increasing irrigation pressure reduces chances of cornea collapse, but causes even higher overpressure peaks during the flow interruption, that could cause damages (detachment) from capsular sac and interruption of blood circulation within the optical nerve head.

These effects are even more meaningful and visible when a high vacuum level is used and, consequently, irrigation flow is high.

The above problem limits vacuum level that can be really used in order to maintain a suitable safety level for procedure, thus reducing efficiency of system and prolonging surgical intervention duration.

Some solutions aiming solving this problem have been suggested. For example, it is possible limiting the aspiration flow with high-vacuum level, statistically or dynamically reducing lumen in a zone of the aspiration line. These systems are subjected to clogging due to nucleus fragments and, furthermore, reduce efficiency of aspiration for reducing flow.

Other solutions acting introducing an elastic device within the irrigation line deforming absorbing overpressure peaks (during the interruption of aspiration due to clogging), sending back liquid accumulated during the re-starting of aspiration (emulsiphication of occlusion), as what is suggested in the solution according to the present invention.

Said devices are inserted within the infusion-aspiration cassette connected with facoemulsiphier (device put on the market by Alcon) at a distance of two meters from eye. Inertia of saline solution column within the irrigation line portion between the infusion-aspiration cassette and the patient eye is not compensated and overpressure and cornea collapse problems are still present at high vacuum levels.

In a further solution, elastic reservoir is inserted within the ultrasound handpiece. This solution, which is optimum as far as flow is concerned, cannot be adopted due to the difficulties for cleaning, decontamination and sterilization of elastic reservoir, as well as for impossibility of inspecting the same and verifying its integrity, which is an important aspect considering that life of the same reservoir will be noticeably lower than the life of handpiece and that, in case device is damaged, serious corneal collapse could occur.

In view of the above, it is suggested by the Applicant the device according to the present invention, permitting solving the above mentioned drawbacks.

US2002/095113 A1 describes a device for stabilization of an eye front chamber during ophthalmic surgery according to the preamble of claim 1.

These and other results are obtained according to the invention by a device for hydrodynamic stabilization of eye front chamber and for preventing collapses of cornea during ophthalmic surgery, comprising a reservoir, with elastic walls, along the irrigation line, connected to the inlet of the ultrasound or Irrigation-Aspiration handpiece irrigation, wherein said elastic reservoir has the function of annulling, or in any case, remarkably reducing effects of inertia opposed to the quick flow variations along irrigation line, causing problems for stability of front chamber, which are typical of the front segment eye surgery, particularly with high vacuum aspiration.

It is therefore specific object of the present invention a device for stabilization of eye front chamber during ophthalmic surgery with high vacuum aspiration, said device having a substantially Y shape, with the first arm of said Y shape in flow communication with an irrigation line arriving from Infusion-Aspiration cassette of phacoemulsiphier, free arm of Y shape in flow communication with said first arm and the second arm of the Y shape figure and realized as a connector for irrigation of an ultrasound handpiece in order to receive a saline solution arriving from the irrigation line in case of clogging of the aspiration line.

According to the invention, said reservoir is comprised of a biocompatible elastic material, preferably biocompatible silicone rubber.

Still according to the invention, said reservoir can have a volume of about 0.5 - 3.0 cc.

The present invention will be now described, for illustrative and not limitative purposes, according to its preferred embodiments, with particular reference to the figures of the enclosed drawings, wherein:
figure 1 shows a graph of intraocular pressure with respect to time for a device according to the prior art;
figure 2 shows a longitudinal section of a device according to the invention; and
figure 3 shows a graph of intraocular pressure with respect to time for a device according to figure 2.

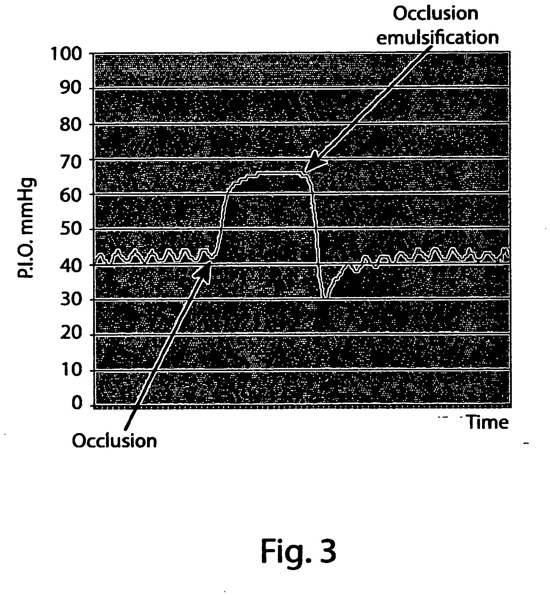

Observing figures 1 and 3 of the enclosed drawings, it is shown a stabilization device according to the invention, generically indicated by reference number 1, providing a small reservoir (A) (0.5 - 3 cc) comprised of elastic material, preferably biocompatible silicone rubber, mounted on an arm 2 of an "Y" shaped joint. A second arm 3 of said Y" shaped joint is connected to the irrigation line arriving from the Infusion-Aspiration cassette (not shown) of phacoemulsiphier. Free arm 4 of the same joint is shaped as a connector and inserts itself directly in connection for irrigation of the ultrasound handpiece.

During a sudden clogging of aspiration line, saline solution arriving from irrigation source, through arm 3 of Y" shaped joint exits within reservoir (A), dilating the same, preventing overpressure within the pressure eye.

When occlusion is removed from phacoemulsifier and aspiration suddenly starts again, reservoir (A) provides small amount of solution saline necessary to limit IntraOcular Pressure drop, until when normal flow of saline solution from irrigation source is established again.

Effects of device according to the invention are clearly evident from figure 3, comparing the same with figure 1.

Preferably, whole device 1 according to the invention is a disposable sterile handpiece, preventing the surgeon mounting and preparing the same and avoiding wearing problems.

With respect to known solutions, and particularly with respect to solution described in the above mentioned US patent n° 6,042,586, it is obtained elimination of latex membrane, which potentially is allergenic, replaced by a silicone reservoir, replacement of membrane laid on a cup with a cylindrical reservoir, that can be manufactured much more easily, reducing volume of device, mainly due to the reservoir shape, that therefore, does not hinder standard surgical operations.

Present invention has been described for illustrative and not limitative purposes with reference to preferred embodiments, but it is understood that variations and/or modifications can be introduced without departing from the relevant scope defined in the enclosed claims.

## Claims

1. Device (1) for stabilization of eye front chamber during ophthalmic surgery with high vacuum aspiration, said device having a substantially Y shape, with the first arm (3) of said Y shape in flow communication with an irrigation line arriving from Infusion-Aspiration cassette of phacoemulsiphier, free arm (4) of Y shape in flow communication with said first arm (3) and the second arm (2) of the Y shape and realized as a connector for irrigation of an ultrasound handpiece, **characterized in that** a reservoir comprised of elastic material is realized in said second arm (2) in order to receive a saline solution arriving from the irrigation line in case of clogging of the aspiration line, said reservoir being comprised of a biocompatible elastic material.

2. Device (1) according to claim 1, **characterized in that** said reservoir is comprised of biocompatible silicone rubber.

3. Device (1) according to claim 1 or 2, **characterized in that** said reservoir has a volume of about 0.5 - 3.0 cc.

## Patentansprüche

1. Einrichtung (1) zur Stabilisierung einer Augen-Frontkammer während einer ophthalmologischen Operation mit Hochvakuum-Aspiration, wobei die Einrichtung eine im Wesentlichen Y-Form aufweist, wobei der erste Arm (3) der Y-Form in Flusskommunikation mit einer Spülleitung ist, die von einer Infusions-Aspirations-Kassette eines Phakoemulsifikator eintrifft, ein freier Arm (4) der Y-Form in Flusskommunikation mit dem ersten Arm (3) und dem zweiten Arm (2) der Y-Form und als ein Konnektor zur Spülung eines Ultraschall-Handstücks realisiert ist, **dadurch gekennzeichnet, dass** ein Reservoir, umfassend elastisches Material, in dem zweiten Arm (2) realisiert ist, um eine Saline-Lösung aufzunehmen, die im Fall eines Zusetzens der Aspirationsleitung von der Spülleitung eintrifft, wobei das Reservoir ein biokompatibles elastisches Material umfasst.

2. Einrichtung (1) gemäß Anspruch 1, **dadurch gekennzeichnet, dass** das Reservoir einen biokompatiblen Silikongummi umfasst.

3. Einrichtung (1) gemäß Anspruch 1 oder 2, **dadurch gekennzeichnet, dass** das Reservoir ein Volumen von ungefähr 0,5 - 3,0 cm³ aufweist.

## Revendications

1. Dispositif (1) pour la stabilisation de la chambre antérieure de l'oeil pendant une chirurgie ophtalmique avec une forte aspiration sous vide, ledit dispositif ayant une forme sensiblement en Y, avec la première branche (3) de ladite forme en Y en communication fluidique avec un conduit d'irrigation provenant de la cassette de perfusion-aspiration du phacoémulsifiant, la branche libre (4) de la forme en Y en communication fluidique avec ladite première branche (3) et la seconde branche (2) de la forme en Y et réalisée en tant que connecteur pour l'irrigation d'une pièce à main à ultrasons, **caractérisé en ce qu'**un réservoir composé d'un matériau élastique est réalisé dans ladite seconde branche (2) afin de recevoir une solution saline provenant du conduit d'irrigation dans le cas d'un engorgement du conduit d'aspiration, ledit réservoir étant composé d'un matériau élastique biocompatible.

2. Dispositif (1) selon la revendication 1, **caractérisé en ce que** ledit réservoir est composé d'un caoutchouc en silicone biocompatible.

3. Dispositif (1) selon la revendication 1 ou 2, **caractérisé en ce que** ledit réservoir a un volume d'environ 0,5 à 3,0 cm³.
